Europäisches Patentamt

European Patent Office

Office européen des brevets

(19)

(11) Veröffentlichungsnummer: **0 322 647 B1**

(12) # EUROPÄISCHE PATENTSCHRIFT

(45) Veröffentlichungstag der Patentschrift: **18.03.92**

(51) Int. Cl.⁵: **C07C 265/00**, C07C 263/10

(21) Anmeldenummer: **88120892.0**

(22) Anmeldetag: **14.12.88**

(54) Verfahren zur kontinuierlichen Herstellung von Mono- oder Polyisocyanaten.

(30) Priorität: **24.12.87 DE 3744001**

(43) Veröffentlichungstag der Anmeldung:
**05.07.89 Patentblatt 89/27**

(45) Bekanntmachung des Hinweises auf die
Patenterteilung:
**18.03.92 Patentblatt 92/12**

(84) Benannte Vertragsstaaten:
**AT BE CH DE ES FR GB IT LI NL**

(56) Entgegenhaltungen:
**GB-A- 2 036 586**
**US-A- 2 563 002**
**US-A- 3 226 410**

(73) Patentinhaber: **BAYER AG**

**W-5090 Leverkusen 1 Bayerwerk(DE)**

(72) Erfinder: **Zaby, Gottfried, Dr.**
**Bergische Landstrasse 124 b**
**W-5090 Leverkusen 1(DE)**
Erfinder: **Judat, Helmut, Dr.**
**Oskar-Erbsloeh-Strasse 44 a**
**W-4018 Langenfeld(DE)**
Erfinder: **Boonstra, Eric**
**3 a Whittengale Road**
**Oakdale, PA 15071(US)**
Erfinder: **De Vos, Stefan**
**Vaartlaan 57**
**B-2128 St. Job(BE)**
Erfinder: **Eckerman, Rolf-W.**
**Wichernstrasse 5**
**W-5060 Bergisch Gladbach(DE)**
Erfinder: **Humburger, Siegbert, Dr.**
**Carl-Rumpfstrasse 8**
**W-5090 Leverkusen 1(DE)**

EP 0 322 647 B1

**Beschreibung**

Die Erfindung betrifft ein Verfahren zur kontinuierlichen Herstellung von organischen Mono- oder Polyisocyanaten durch Umsetzung der den Mono- oder Polyisocyanaten entsprechenden Mono- oder Polyamine mit in organischem Lösungsmittel gelöstem Phosgen bei erhöhten Temperaturen.

Es ist bekannt, derartige Reaktionsgemische aus organischen Aminlösungen und organischen Phosgenlösungen in Mischern mit beweglichen Teilen, wie beispielsweise in einem Kreiselpumpenmischer (DE-AS 2 153 268 oder US-PS 3 947 484) herzustellen. Wegen der Toxizität von Phosgen stellen insbesondere Wellendurchführungen bei derartigen Mischern Gefahrenquellen durch Leckage dar. Da bei der Reaktion auch Feststoffe entstehen, sind Anbackungen oft nicht zu vermeiden.

Man hat deshalb auch schon nach Verfahren gesucht, bei welchen die Vermischung ohne bewegte Teile stattfindet: Gemäß DE-OS 29 50 216 läßt man die beiden Reaktionskomponenten in einer zylindrischen Mischkammer in Form fächerartiger Spritzstrahlen aufeinanderprallen. Bei diesem Verfahren bedarf es hoher Vordrücke und wegen der strömungstoten Winkel der Mischkammer kann es zu Verstopfungen kommen.

Schließlich ist es bekannt (US-PS 3 226 410), die Aminlösung durch seitlich in einem Rohr angebrachte Bohrungen in die im Rohr strömende Phosgenlösung einzuspritzen. Bei der zur Erzielung akzeptabler Ausbeuten erforderlichen geringen Konzentration der Reaktanden ist auch die produzierte Isocyanatmenge im Verhältnis zur Lösungsmittelmenge entsprechend niedrig. Der dadurch bedingte hohe Energieeinsatz zur Lösungsmittel-Rückgewinnung ist unbefriedigend. Ein Schichtaufbau aus Feststoffen an der Wandung läßt sich nicht immer vermeiden.

Bei den bekannten Verfahren muß im übrigen unter starker Verdünnung der Reaktanten gearbeitet werden. Dies und die durch häufigen Verstopfungen verursachten Stillstandzeiten für die Reinigung der Anlagen führt zu einer negativen Beeinträchtigung der Wirtschaftlichkeit der bekannten Verfahren.

Es bestand somit die Aufgabe, ein neues Verfahren zur kontinuierlichen Herstellung von organischen Mono- oder Polyisocyanaten zur Verfügung zu stellen, bei welchem die Menge des eingesetzten Hilfslösungsmittels deutlich reduziert werden kann und dennoch Schwierigkeiten bezüglich der Bildung von Feststoffanbackungen und Verstopfungen weitgehend vermieden werden. Außerdem sollte das neue Verfahren auf die Verwendung von beweglichen Teilen verzichten, so daß bezüglich der Sicherheit des Verfahrens (Toxizität) die obengenannten Probleme nicht auftreten können.

Diese Aufgabe wurde erfindungsgemäß dadurch gelöst, daß man zur Herstellung des Reaktionsgemischs die gegebenenfalls gelöste Aminkomponente mit der Phosgenlösung in einer speziellen Düse der nachstehend näher beschriebenen Art zusammenführt.

Gegenstand der Erfindung ist ein Verfahren zur kontinuierlichen Herstellung von organischen Mono- oder Polyisocyanaten durch Umsetzung der den Mono- oder Polyisocyanaten entsprechenden Mono- oder Polyamine mit in organischem Lösungsmittel gelöstem Phosgen bei erhöhten Temperaturen und anschließende destillative Aufarbeitung des resultierenden Reaktionsgemischs, dadurch gekennzeichnet, daß man zur Herstellung der Ausgangsgemische die gegebenenfalls in einem inerten Lösungsmittel gelöste Aminkomponente und die Phosgenlösung in einer Düse (1) zusammenführt, indem die eine der beiden Komponenten in dieser Düse (1) eingeschnürt wird und die andere Komponente in dieser Einschnürung (3) dem Strom der ersten Komponente in mehreren Teilströmen durch eine entsprechende Anzahl von über den Umfang der Einschnürung (3) verteilten Bohrungen (5) von der Seite her zugeführt wird, daß in der Einschnürung eine Strömungsgeschwindigkeit von 1 bis 10 m/sec eingehalten wird, daß die gesamte Länge L der Einschnürung (3) mindestens dem 2-fachen Durchmesser D der Einschnürung (3) entspricht und für die Lange $L_1$ der Einschnürung (3) des axial zugeführten Stromes bis zur Zusammenführung mit den Teilströmen der zweiten Komponente das 0,5- bis 2-fache des Durchmessers D der Einschnürung (3) gewählt wird, daß der aus den beiden Strömen resultierende Produktstrom einer konstant bleibenden Einschnürung (3) unterworfen wird, deren Länge $L_2$ mindestens jener Strecke entspricht, in welcher die Reaktion des freien Amins im wesentlichen abgeschlossen ist, daß das Leistungsverhältnis des axial zugeführten Stromes $\epsilon_A$ zum seitlich zugeführten Strom $\epsilon_S$

$$\frac{\epsilon_A}{\epsilon_S} = \frac{\varrho_A \cdot \overset{\circ}{V}_A \cdot v_A{}^2}{\varrho_S \cdot \overset{\circ}{V}_S \cdot v_S{}^2} = 0,01 \text{ bis } 1,0,$$

eingehalten wird, wobei

2

$\rho$     die Dichte,

$\overset{\lor}{V}$     den Volumenstrom,

v     die Strömungsgeschwindigkeit,

und zwar jeweils mit dem Index A des axial zugeführten Stromes und jeweils mit dem Indes S des seitlich zugeführten Stromes,

bedeuten,

daß hinter der Einschnürung (3) der Strömungsquerschnitt stetig erweitert wird und daß die Anzahl i der Bohrungen (5) für die seitlich zuzuführenden Teilströme zwischen 2 ≦ i ≦ m gewählt wird, wobei sich m aus der Bedingung

$$\frac{\pi \cdot D}{m \cdot d} = \rangle\ 1,1\ \text{ergibt,}$$

wobei D den Durchmesser der Einschnürung (3) und d den Durchmesser der Bohrungen (5) bedeuten.

Es hat sich überraschenderweise gezeigt, daß bei diesem Verfahren mit niedrigen Druckverlusten und höheren Konzentrationen gearbeitet werden kann, daß eine hohe Produktausbeute bei sehr geringen Verweilzeiten in den dem Mischaggregat nachgeschalteten Reaktionsräumen erzielbar ist und daß insbesondere die Verstopfungen und Anbackungen vermieden werden. Die verwendete Düse wird auch als Ringlochdüse bezeichnet. Die Einschnürung des Lösungsstromes kann sprunghaft oder stetig erfolgen. Vorzugsweise erfolgt jedoch die Einschnürung durch eine plötzliche Verengung der Zuleitung. Da in der Ringlochdüse zur Erzielung optimaler Vermischung in der Regel nur ein Druckverlust von etwa 2 bar erforderlich ist, können die Vordrücke der Lösungsströme in der Regel klein gehalten werden. Dadurch können die bisher üblichen Pumpen eingesetzt werden. Es können jedoch auch höhere Druckverluste zur Anwendung gelangen, wenn der Nachteil des höheren Vordrucks in Kauf genommen wird.

Als Ausgangsmaterialien kommen als organische Amine aliphatische, cycloaliphatische, aliphatisch-aromatische, aromatische Amine, Di- und/oder Polyamine in Frage, wie z.B. Anilin, Halogen-substituierte Phenylamine, wie 4-Chlorphenylamin, 1,6-Diaminohexan, 1-Amino-3,3,5-trimethyl-5-aminomethyl-cyclohexan, 2,4-Diaminotoluol, dessen technische Gemische mit 2,6-Diaminotoluol, die im allgemeinen bis zu 35 Gew.-%, bezogen auf Gemisch, an 2,6-Diaminotoluol enthalten oder Polyamingemische der Diphenylmethanreihe, wie sie durch an sich bekannte Anilin-Formaldehyd-Kondensation zugänglich sind. Weiterhin können beispielsweise die in US-PS 3 321 283, Kolonne 4, Zeile 19-61 genannten Amine zum Einsatz gelangen.

Die zu phosgenierenden Amine können beim erfindungsgemäßen Verfahren in Substanz, d.h. ohne inertes Lösungsmittel eingesetzt werden. Die Aminkomponente kann jedoch auch in in inertem Lösungsmittel gelöster Form zur Anwendung gelangen, wobei beliebige Konzentrationen denkbar sind. Eine hohe Aminkonzentration bringt einerseits zu Energieeinsparungen bei der Lösungsmittelrückgewinnung und kann andererseits zu geringen Ausbeuteverlusten führen. Diese Vor- und Nachteile müssen gegeneinander abgewogen werden. Oftmals werden die Amine in 5- bis 50-, vorzugsweise 5- bis 40 und insbesondere 10- bis 25-gew.-%iger Konzentration in Form von Lösungen in inerten Lösungsmitteln verwendet. Bevorzugte Lösungsmittel sind chlorierte, aromatische Kohlenwasserstoffe, wie beispielsweise Chlorbenzol, o-Dichlorbenzol, p-Dichlorbenzol, Trichlorbenzole, die entsprechenden Chlortoluole und -xylole, Chlorethylbenzol, Monochlordiphenyl, $\alpha$-bzw, $\beta$-Naphthylchlorid, Benzoesäureethylester, Phthalsäuredialkylester, Diiso-diethylphthalat, Toluol und Xylole. Die Lösungsmittel können einzeln oder als Gemische verwendet werden. Weitere geeignete Lösungsmittel sind beispielsweise in US-PS 3 321 283, Kolonne 4, Zeile 66 bis Kolonne 5, Zeile 2 beschrieben.

Das Phosgen kommt beim erfindungsgemäßen Verfahren im allgemeinen in Form von 10- bis 85-, vorzugsweise 40-bis 70- und insbesondere 40- bis 65-gew.-%igen, Lösungen in inerten Lösungsmitteln zum Einsatz, wobei vorzugsweise für das Phosgen das gleiche Lösungsmittel wie für das Amin verwendet wird. Die Phosgenlösungen können auch im Kreislauf geführtes Verfahrensprodukt (Mono- oder Polyisocyanat) enthalten. Falls die Phosgenlösung Verfahrensprodukt enthält, muß auf jeden Fall ein hoher Phosgenüberschuß vorliegen, damit die Bildung von Harnstoffen (Umsetzung der primären Amine mit dem Isocyanat) weitgehend vermieden wird. Dies bedeutet, daß im Falle der Verwendung von Phosgenlösungen, die Mono- oder Polyisocyanate enthalten, stets ein mindestens 2,5-fach molarer Überschuß an Phosgen gegenüber Isocyanatgruppen in den Lösungen vorliegen muß.

Das Äquivalentverhältnis von Phosgen zu Amin liegt im allgemeinen bei mindestens 1,5 : 1, in Sonderfällen bis zu 20 : 1, vorzugsweise bei 1,5 : 1 bis 7 : 1 und insbesondere bei 2 : 1 bis 5 : 1.

Zur erfindungsgemäßen Herstellung der Reaktionsgemische unter Verwendung des erfindungswesentlichen Mischaggregats kann beispielsweise wie folgt verfahren werden:

Die Temperatur der Phosgenlösung sollte unterhalb der Siedetemperatur der Lösung liegen, dies bedeutet, es werden im allgemeinen entsprechend gekühlte Phosgenlösungen verwendet. Die Temperatur der Aminkomponente hängt im allgemeinen von den physikalischen Eigenschaften des Amins, d.h. vom Schmelzpunkt des Amins bzw. vom Kristallisationspunkt der zum Einsatz gelangenden Aminlösung ab und kann innerhalb von weiten Bereichen schwanken.

Vorzugsweise wird die Lösung mit dem größeren Volumenstrom durch die Einschnürung (3) geführt, wobei im Falle der Verwendung von annähernd gleichen Volumenströmen jede der beiden Komponenten den Mittel- bzw. Seitenstrom bilden kann. Die Beachtung dieser Maßnahmen dient einer optimalen Vermischung und damit einem einwandfreien Reaktionsablauf.

In der Einschnürung wird im Rahmen der Erfindung eine Strömungsgeschwindigkeit von 1 bis 10 m/sec eingehalten. Daneben können jedoch auch höhere Strömungsgeschwindigkeiten in der Einschnürung beispielsweise bis zu 50 m/sec eingehalten werden, wenn der Nachteil des hierzu erforderlichen hohen Pumpenvordrucks in Kauf genommen wird. Umgekehrt kann selbstverständlich bei der Strömungsgeschwindigkeit von 1 bis 10 m/sec der Vordruck in vorteilhafter Weise gering gehalten werden, Die Strömungsgeschwindigkeit in der Einschnürung beträgt im allgemeinen das 2- bis 50-fache, vorzugsweise das 2- bis 10-fache der Strömungsgeschwindigkeit im Zuleitungsrohr zu der Einschnürung.

Als Länge L wird mindestens der 2-fache Durchmesser D der Einschnürung gewählt.

Diese Ausgestaltung dient einer besonders intensiven Vermischung und die Strömung stabilisiert sich in ausreichender Weise.

Für die Länge $L_1$ der Einschnürung des axial zugeführten Stromes bis zur Zusammenführung mit den Teilströmen der zweiten Komponente wird das 0,5- bis 2-fache des Durchmessers D der Einschnürung gewählt.

Im neuen Verfahren wird der aus beiden Strömen resultierende Produktstrom einer konstant bleibenden Einschnürung unterworfen, deren Länge $L_2$ mindestens jener Strecke entspricht, in welcher die Reaktion des freien Amins im wesentlichen abgeschlossen ist, Die Länge $L_2$ beträgt im allgemeinen maximal das 2-fache des Durchmessers D. Größere Abmessungen $L_2$ führen zu höheren Druckverlusten, ohne das damit irgendein Vorteil verbunden wäre,

Die genannten Maßnahmen stellen sicher, daß sich keine Anbackungen in der Düse bilden.

Eine besonders hohe Ausbeute läßt sich erzielen, indem ein Leistungsverhältnis des axial zugeführten Stromes $\epsilon_A$ zum seitlich zugeführten Strom $\epsilon_S$

$$\frac{\epsilon_A}{\epsilon_S} = \frac{\varrho_A \cdot \overset{o}{V}_A \cdot v_A^2}{\varrho_S \cdot \overset{o}{V}_S \cdot v_S^2} = 0{,}01 \text{ bis } 3{,}0,$$

vorzugsweise von 0,01 bis 1,0 und besonders bevorzugt von 0,05 bis 0,7, eingehalten wird, wobei

$\rho$ die Dichte ($kg/m^3$),

$\overset{o}{V}$ den Volumenstrom ($m^3/sec$),

$v$ die Strömungsgeschwindigkeit (m/sec) und zwar jeweils mit dem Index A des axial zugeführten Stromes und jeweils mit dem Index S des seitlich zugeführten Stromes bedeuten.

Die hiermit erzielbare hohe Ausbeute gewährleistet gleichzeitig einen niedrigen Energieeinsatz.

Durch die stetige Erweiterung des Strömungsquerschnitts hinter der Einschnürung wird erreicht, daß keine Verwirbelung und Rückströmung stattfinden können. Es versteht sich, daß die Erweiterung bei einem Maximum endet, welches dem Durchmesser einer anschließenden Rohrleitung entspricht. Die Vermeidung einer Rückströmung bietet eine besondere Gewähr für die Vermeidung von Anbackungen und Verstopfungen.

Die Anzahl i der Bohrungen für die seitlich zuzuführenden Teilströme wird zwischen $2 \leq i \leq m$, vorzugsweise zwischen $6 \leq i \leq m$, gewählt, wobei sich m aus der Bedingung

$$\frac{\pi \cdot D}{m \cdot d} = \;\rangle\; 1,1, \text{ vorzugsweise } \rangle\; 1,5, \text{ insbesondere}$$

$\geq 2$ ergibt, wobei D den Durchmesser der Einschnürung und d den Durchmesser der Bohrungen bedeuten.

Diese Maßnahme wirkt sich ebenfalls günstig auf die Vermischung und damit auf die Reaktion sowie auf die Vermeidung von Anbackungen aus.

Vorzugsweise werden alle Bohrungen in einer gemeinsamen Ebene senkrecht zur Einschnürung angeordnet, obwohl auch Abweichungen hiervon möglich sind.

Dadurch wird erreicht, daß die Reaktion nur in dieser Ebene starten kann, also bereits reagierendes Produkt nicht mehr erneut mit der zweiten Komponente beaufschlagt wird. Hierdurch wird die Ausbeute erhöht und die Gefahr von Anbackungen herabgesetzt, wodurch die Wirtschaftlichkeit des Verfahrens erhöht wird.

Da die Phosgenlösung im allgemeinen den größeren Volumenstrom darstellt, wird sie, den oben gemachten Ausführungen entsprechend, im allgemeinen durch die Einschnürung geleitet.

Die Zeichnungen dienen zur näheren Erläuterung des erfindungswesentlichen Mischaggregats.

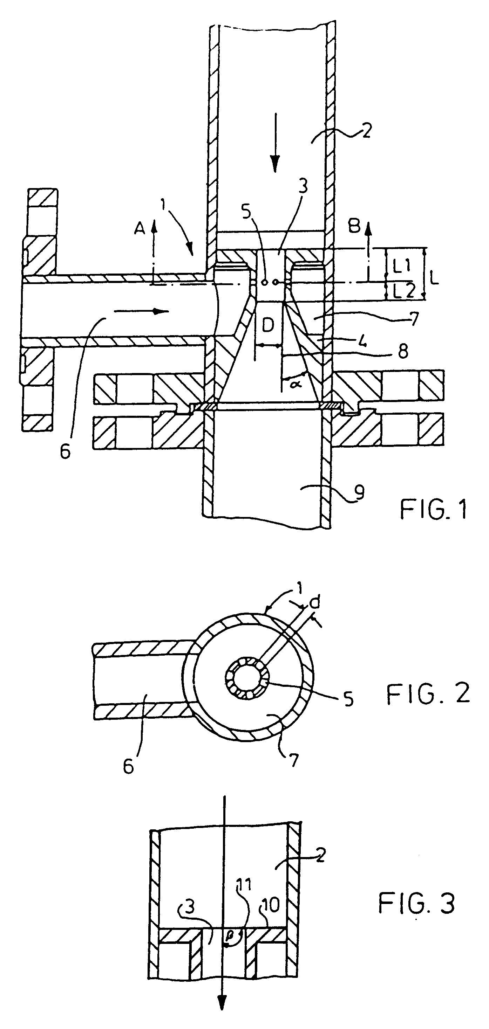

Fig. 1 zeigt die Düse im Längsschnitt,

Fig. 2 einen Schnitt gemäß Linie A-B in Fig. 1 und

Fig. 3 den Übergang des Zuleitungsrohrs in die Einschnürung

Im einzelnen bedeuten:

(1) die erfindungsgemäß zu verwendende Düse;

(2) das Zuleitungsrohr für den Hauptstrom;

(3) die sprunghafte Einschnürung des Hauptstroms;

(4) den die Einschnürung verursachenden Einsatz mit eingebauten Seitenbohrungen;

(5) die Seitenbohrungen;

(6) die Zuleitung des Seitenstroms;

(7) die die Einschnürung (3) umgebende Kammer, aus welcher die Bohrungen (5) abführen;

(8) die stetige Erweiterung am Ausgang der Düse;

(9) das Abführrohr;

(10) die Staufläche am Ende des Zuleitungsrohrs (2);

(11) die Abrißkante am Beginn der Einschnürung (3);

D den Innendurchmesser der Einschnürung;

d den Durchmesser der Seitenbohrungen;

L die Gesamtlänge der Einschnürung;

$L_1$ den Abstand vom Beginn der Einschnürung bis zur Ebene der Seitenbohrungen und

$L_2$ den Abstand von der Ebene der Seitenbohrungen bis zum Beginn der stetigen Erweiterung.

Zur Herstellung der Reaktionsmischungen unter Verwendung des erfindungswesentlichen Mischaggregats kann beispielsweise wie folgt vorgegangen werden:

Einer Düse 1 wird über ein Zuleitungsrohr 2, welches sprunghaft an der Staufläche 10 über die Abrißkante 11 in eine in der Düse 1 angeordnete Einschnürung 3 übergeht, der Hauptstrom zugeführt. Die Staufläche 10 bildet vorzugsweise mit der Strömungsrichtung einen Winkel $\beta$ von $90 \pm 45°$, der, wie leicht ersichtlich, dem Winkel der Abrißkante 11 entspricht. Die Einschnürung 3 ist in einem Einsatz 4 angeordnet. Die Einschnürung 3 weist über ihre gesamte Länge L den konstanten Durchmesser D auf und im Abstand $L_1$, welcher z.B. dem 1,5-fachen Durchmesser D der Einschnürung 3 entspricht, sind z.B. sechs Bohrungen 5 über dem Umfang regelmäßig verteilt angeordnet. Einander gegenüberliegende Bohrungen 5 sind um den Durchmesser d gegeneinander versetzt, so daß die eingespritzten Teilströme aneinander vorbeizielen. Die zweite Komponente wird über ein Zuleitungsrohr 6 einer die Einschnürung 3 umgebenden Kammer 7, aus welcher die Bohrungen 5 abführen, zugeführt. Die Länge $L_2$ der Einschnürung 3 hinter den Bohrungen 5 entspricht z.B. dem Durchmesser D der Einschnürung 3 und damit in etwa demjenigen Bereich, in welchem die Reaktion des freien Amins im wesentlichen abgeschlossen ist. Hinter der Einschnürung 3 weist die Düse 1 eine stetige Erweiterung 8 auf, deren Winkel $\alpha$ mit der Achse z.B. $20°$ beträgt. An diese Erweiterung 8 schließt sich ein Abführrohr 9 gleichen Durchmessers wie das Zuleitungsrohr 2 an.

Die unter Verwendung des erfindungswesentlichen Mischaggregats hergestellten Reaktionsgemische können im Anschluß an ihre Herstellung in den üblichen Reaktoren, wie beispielsweise Rührkesseln oder Phosgeniertürmen zum gewünschten Verfahrensprodukt, d.h. zum Mono- oder Polyisocyanat ausreagiert werden. Die zum Verfahrensprodukt führende chemische Reaktion wird im allgemeinen innerhalb des

EP 0 322 647 B1

Temperaturbereichs von 20 bis 180°C durchgeführt.

Eine besonders bevorzugte Ausführungsform des erfindungsgemäßen Verfahrens besteht darin, das unter Verwendung des erfindungswesentlichen Mischaggregats hergestellte Reaktionsgemisch von unten nach oben durch eine Reaktionskolonne zu leiten, die mit Lochböden ausgerüstet ist, so daß der Innenraum der Kolonne in mindestens 10, vorzugsweise 20 bis 50, durch die horizontal eingebauten Lochböden voneinander abgetrennten Kammern eingeteilt ist. Grundsätzlich möglich, jedoch keinesfalls bevorzugt, wäre auch die Verwendung von mehreren in Serie geschalteten Lochbodenkolonnen mit insgesamt mindestens 10, vorzugsweise 20 bis 50 Kammern.

Eine noch weitergehende Kammerung ist nicht sinnvoll, weil sich einerseits unter jedem Lochboden ein Gaspolster ausbildet, das den festen und flüssigen Bestandteilen des Reaktionsgemisches als Reaktionsraum verloren geht, und andererseits die zusätzliche Verbesserung des Verweilzeitspektrums minimal ist.

Die Löcher der Lochböden weisen im allgemeinen einen Durchmesser von maximal 20 mm, vorzugsweise von 2 bis 10 mm auf, wobei die Anzahl der Bohrungen in Abhängigkeit vom Durchsatz vorzugsweise so gewählt wird, daß eine Rückvermischung des von unten nach oben aufsteigenden Reaktionsgemischs zwischen den einzelnen Kammern weitgehend verhindert wird.

Bei dem in der Kolonne aufsteigenden Reaktionsgemisch handelt es sich um ein Gemisch aus flüssigen Bestandteilen (Lösungen der Ausgangsmaterialien und der sich bildenden Isocyanate), gasförmigen Bestandteilen (Phosgen und sich bildender Chlorwasserstoff) und (zumindest zu Beginn der Reaktion) festen Bestandteilen (im Lösungsmittel suspendierte Carbamoylchloride bzw. Amin-hydrochloride). Optimale Reaktionsbedingungen liegen dann vor, wenn die Geschwindigkeit der aufsteigenden Gasphase in den Löchern der Lochböden bei 2 bis 20, vorzugsweise 3,5 bis 10 m/sec und die Geschwindigkeit der aufsteigenden flüssigen Phase in den Löchern der Lochböden bei 0,05 bis 0,4, vorzugsweise 0,1 bis 0,2 m/sec liegen.

Die Temperatur des, die Mischapparatur verlassenden, Reaktionsgemischs liegt im allgemeinen im Falle der bevorzugten Arbeitsweise unter Verwendung von Lochbodenkolonnen bei 40 bis 100°C, während die Temperatur am Kopf der Reaktionskolonne unterhalb 150°C, vorzugsweise bei 70 bis 130, insbesondere bei 90 bis 125°C liegt. Dies wird im allgemeinen durch geeignete Beheizung der Reaktionskolonne erreicht. Um das notwendige Phosgeniervolumen zu minimieren, ist es vorteilhaft, die zum Erreichen der gewünschten Überlauftemperatur erforderliche Energie im unteren Bereich des Phosgenierturms oder auch bereits vor Eintritt in den Reaktor einzubringen. Dadurch wird vermieden, daß ein Teil des Reaktorvolumens wegen zu niedriger Temperatur und daher zu langsamer Bruttoreaktionsgeschwindigkeit ineffektiv ist.

Die Abmessungen der Reaktionskolonne, sowie die Konstruktion der Böden und die Menge des in die Kolonne unten eingebrachten Reaktionsgemischs werden im übrigen im allgemeinen so bemessen, daß die mittlere Verweilzeit des Reaktionsgemisches in der Reaktionskolonne bei maximal 120, vorzugsweise maximal 60 Minuten liegt.

Der Druck am Kopf der Reaktionskolonne liegt im allgemeinen bei 1,2 bis 3, vorzugsweise 1,5 bis 2,5 bar (abs.). Es kann jedoch auch unter höheren oder niedrigeren Drücken gearbeitet werden.

Das die Reaktionskolonne am oberen Ende verlassende, flüssige und gasförmige Bestandteile enthaltende Reaktionsgemisch wird in an sich bekannter Weise zunächst von gasförmigen Bestandteilen (überschüssiges Phosgen und Chlorwasserstoff) befreit und anschließend destillativ aufgearbeitet. Auch bei Verwendung der obengenannten klassischen Reaktionsgefäße zur Durchführung der Phosgenierungsreaktion erfolgt selbstverständlich im Anschluß an die chemische Reaktion eine destillative Aufarbeitung des Reaktionsgemischs. Vor dieser destillativen Aufarbeitung kann jedoch ein Teil des in Form einer Lösung vorliegenden Reaktionsgemisch mit frischer Phosgenlösung angereichert und an den Prozeßanfang zurückgeführt werden.

In den nachfolgenden Beispielen beziehen sich alle Prozentangaben auf Gewichtsprozente. In allen Beispielen wurden Ringlochdüsen verwendet, bei welchen die Einschnürung (3) durch plötzliche Verengung der Zuleitung (2) erfolgte, wobei die Staufläche (10) mit der Strömungsrichtung einen Winkel $\beta$ von 90° bildet.

Beispiel 1

In einer Ringlochdüse mit einem Durchmesser D von 14 mm und einer Länge L von 28 mm ($L_1$ = 18 mm) der Einschnürung und 10 Bohrungen von 2,1 mm Durchmesser am Umfang der Einschnürung wurde eine Lösung aus 550 kg/h 3-Chlor-4-methyl-phenylaminund 650 kg/h Monochlorbenzol (MCB) mit 3240 kg/h einer 50 %igen Phosgen-Lösung in MCB vermischt, wobei die Aminlösung durch die 10 Seitenbohrungen zugeführt wurde. Die Strömungsgeschwindigkeit der Phosgen-Lösung in der Einschnürung betrug 4,9 m/s, diejenige der Aminlösung in den Seitenbohrungen 9,2 m/s. Das Leistungsverhältnis der beiden Lösungen betrug $\epsilon_A/\epsilon_S$ = 0,75. Das Reaktionsgemisch wurde anschließend in einer 3-Kessel-Kaskade, in der jeder Kessel ein Volumen von 6 m$^3$ aufwies, bei Temperaturen von 80, 110 bzw. 140°C klarphosgeniert und destillativ aufgearbeitet.

Ausbeute: 98,0 %.

Beispiel 2

In einer Ringlochdüse mit einem Durchmesser D von 19 mm und einer Länge L von 38 mm (L$_1$ = 28,5 mm) der Einschnürung und 12 Bohrungen von 2,6 mm Durchmesser am Umfang der Einschnürung wurde eine Lösung aus 450 kg/h Hexamethylendiamin (HDA) und 4050 kg/h o-Dichlorbenzol (ODB) mit 9000 kg/h einer 30 %igen Phosgen-Lösung in ODB vermischt, wobei die Aminlösung durch die 12 Seitenbohrungen zugeführt wurde. Die Strömungsgeschwindigkeit der Phosgen-Lösung in der Einschnürung betrug 6,5 m/s, diejenige der Aminlösung in den Seitenbohrungen 16,4 m/s.

Das Leistungsverhältnis der beiden Lösungen betrug $\epsilon_A/\epsilon_S$ = 0,32. Das Reaktionsgemisch wurde anschließend in einer mit 45 Lochböden ausgestatteten Reaktionskolonne von 17 m$^3$ Inhalt bei Temperaturen bis 150°C klarphosgeniert und destillativ aufgearbeitet.

Ausbeute: 96 %.

Beispiel 3

In einer Ringlochdüse mit einem Durchmesser D von 10 mm und einer Länge L von 20 mm (L$_1$ = 15 mm) der Einschnürung und 4 Bohrungen von 1,5 mm Durchmesser am Umfang der Einschnürung wurde eine Lösung aus 120 kg/h Trimethylhexamethylendiamin (TMDA) und 145 kg/h Monochlorbenzol (MCB) mit 2835 kg/h einer 50 %igen Phosgen-Lösung in MCB vermischt, wobei die Aminlösung durch die 4 Seitenbohrungen zugeführt wurde. Die Strömungsgeschwindigkeit der Phosgen-Lösung in der Einschnürung betrug 5,7 m/s, diejenige der Aminlösung in den Seitenbohrungen 11,6 m/s. Das Leistungsverhältnis der beiden Lösungen betrug $\epsilon_A/\epsilon_S$ = 1,92. Das Reaktionsgemisch wurde anschließend in einer 3-Kessel-Kaskade, in der jeder Kessel ein Volumen von 6 m$^3$ aufwies, bei Temperaturen von 80, 110 bzw. 140°C klarphosgeniert und destillativ aufgearbeitet.

Ausbeute: 94 %.

Beispiel 4

In einer Ringlochdüse mit einem Durchmesser D von 20 mm und einer Länge L von 36 mm (L$_1$ = 26 mm) der Einschnürung von 20 mm und 12 Bohrungen von 2,6 mm Durchmesser am Umfang der Einschnürung wurde eine Lösung aus 450 kg/h 2,4-Toluylendiamin (TDA) und 2360 kg/h o-Dichlorbenzol (ODB) mit 7300 kg/h einer 50 %igen Phosgen-Lösung in ODB vermischt, wobei die Aminlösung durch die 12 Seitenbohrungen zugeführt wurde. Die Strömungsgeschwindigkeit der Phosgen-Lösung in der Einschnürung betrug 4,8 m/s, diejenige der Aminlösung in den Seitenbohrungen 10,3 m/s. Das Leistungsverhältnis der beiden Lösungen betrug $\epsilon_A/\epsilon_S$ = 0,55. Das Reaktionsgemisch wurde anschließend in einer mit 23 Lochböden ausgestatteten Reaktionskolonne von 7 m$^3$ Inhalt bei Temperaturen bis ca. 100°C klarphosgeniert und destillativ aufgearbeitet.

Ausbeute: 96,7 %.

Beispiel 5

In einer Ringlochdüse mit einem Durchmesser D von 20 mm und einer Länge L von 36 mm (L$_1$ = 26 mm) der Einschnürung und 12 Bohrungen von 2,2 mm Durchmesser am Umfang der Einschnürung wurde eine Lösung aus 550 kg/h eines Gemisches aus 65 % 2,4-Toluylendiamin (2,4-TDA) und 35 % 2,6-Toluylendiamin (2,6-TDA) und 2500 kg/h o-Dichlorbenzol (ODB) mit 6160 kg/h einer 58 %igen Phosgen-Lösung in ODB vermischt, wobei die Aminlösung durch die 12 Seitenbohrungen zugeführt wurde. Die Strömungsgeschwindigkeit der Phosgen-Lösung in der Einschnürung betrug 4,0 m/s, diejenige der Aminlösung in den 12 Seitenbohrungen 15,7 m/s. Das Leistungsverhältnis der beiden Lösungen betrug $\epsilon_A/\epsilon_S$ = 0,13. Das Reaktionsgemisch wurde anschließend in einer mit 23 Lochböden ausgestatteten Reaktionskolonne von 7 m$^3$ Inhalt bei Temperaturen bis ca. 100°C klarphosgeniert und destillativ aufgearbeitet.

Ausbeute: 97 %.

Beispiel 6

In einer Ringlochdüse mit einem Durchmesser D von 23 mm und einer Länge L von 40,2 mm ($L_1$ = 30,2 mm) der Einschnürung und 12 Bohrungen von 3,7 mm Durchmesser am Umfang der Einschnürung wurde eine Lösung aus 1000 kg/h eines Polyamingemisches der Diphenylmethanreihe (MDA, Zweikernanteil ca. 65 %, Viskosität 55 cP bei 80°C) und 4000 kg/h o-Dichlorbenzol (ODB) mit 7140 kg/h einer 45 %igen Phosgen-Lösung in ODB vermischt, wobei die Aminlösung durch die 12 Seitenbohrungen zugeführt wurde. Die Strömungsgeschwindigkeit der Phosgen-Lösung in der Einschnürung betrug 3,5 m/s, diejenige der Aminlösung in den 12 Seitenbohrungen 9,2 m/s. Das Leistungsverhältnis der beiden Lösungen betrug $\epsilon_A/\epsilon_S$ = 0,20. Das Reaktionsgemisch wurde in zwei hintereinander geschalteten Reaktionskolonnen, die jeweils mit 23 Lochböden ausgerüstet waren und einen Inhalt von 7 bzw. 3,5 m$^3$ hatten, bei Temperaturen bis 85°C in der ersten und 155°C in der zweiten Kolonne aufphosgeniert. Nach destillativer Entfernung des Lösungsmittels betrug die Viskosität der lösungsmittelfreien Rohware 45 mPa.s bei 25°C.
Ausbeute: 100 %.

**Patentansprüche**

1. Verfahren zur kontinuierlichen Herstellung von organischen Mono-oder Polyisocyanaten durch Umsetzung der den Mono-oder Polyisocyanaten entsprechenden Mono- oder Polyamine mit in organischem Lösungsmittel gelöstem Phosgen bei erhöhten Temperaturen und anschließende destillative Aufarbeitung des resultierenden Reaktionsgemischs, dadurch gekennzeichnet, daß man zur Herstellung der Ausgangsgemische die gegebenenfalls in einem inerten Lösungsmittel gelöste Aminkomponente und die Phosgenlösung in einer Düse (1) zusammenführt, indem die eine der beiden Komponenten in dieser Düse (1) eingeschnürt wird und die andere Komponente in dieser Einschnürung (3) dem Strom der ersten Komponente in mehreren Teilströmen durch eine entsprechende Anzahl von über den Umfang der Einschnürung (3) verteilten Bohrungen (5) von der Seite her zugeführt wird,
daß in der Einschnürung eine Strömungsgeschwindigkeit von 1 bis 10 m/sec eingehalten wird, daß die gesamte Länge L der Einschnürung (3) mindestens dem 2-fachen Durchmesser D der Einschnürung (3) entspricht und für die Länge $L_1$ der Einschnürung (3) des axial zugeführten Stromes bis zur Zusammenführung mit den Teilströmen der zweiten Komponente das 0,5- bis 2-fache des Durchmessers D der Einschnürung (3) gewählt wird, daß der aus den beiden Strömen resultierende Produktstrom einer konstant bleibenden Einschnürung (3) unterworfen wird, deren Lange $L_2$ mindestens jener Strecke entspricht, in welcher die Reaktion des freien Amins im wesentlichen abgeschlossen ist, daß das Leistungsverhältnis des axial zugeführten Stromes $\epsilon_A$ zum seitlich zugeführten Strom $\epsilon_S$

$$\frac{\epsilon_A}{\epsilon_S} = \frac{\rho_A \cdot \overset{\circ}{V}_A \cdot v_A^2}{\rho_S \cdot \overset{\circ}{V}_S \cdot v_S^2} = 0,01 \text{ bis } 1,0,$$

eingehalten wird, wobei
$\rho$     die Dichte,
$\overset{\circ}{V}$     den Volumenstrom,
$v$     die Strömungsgeschwindigkeit, und zwar jeweils mit dem Index A des axial zugeführten Stromes und jeweils mit dem Indes S des seitlich zugeführten Stromes,
bedeuten,
daß hinter der Einschnürung (3) der Strömungsquerschnitt stetig erweitert wird und daß die Anzahl i der Bohrungen (5) für die seitlich zuzuführenden Teilströme zwischen $2 \leq i \leq m$ gewählt wird, wobei sich m aus der Bedingung

$$\frac{\pi \cdot D}{m \cdot d} = \rangle \, 1,1 \text{ ergibt,}$$

wobei D den Durchmesser der Einschnürung (3) und d den Durchmesser der Bohrungen (5) bedeuten.

2. Verfahren nach Anspruch 1, dadurch gekennzeichnet, daß der größere Volumenstrom durch die Einschnürung (3) geführt wird.

3. Verfahren nach Anspruch 1 oder 2, dadurch gekennzeichnet, daß die Einschnürung (3) über ihre gesamte Länge L einen konstanten Durchmesser D aufweist.

4. Verfahren nach einem der Ansprüche 1 bis 3, dadurch gekennzeichnet, daß man das durch Zusammenführung der Aminkomponente mit der Phosgenlösung erhaltene Reaktionsgemisch bei erhöhten, jedoch unterhalb 150°C liegenden Temperaturen kontinuierlich von unten nach oben eine Reaktionskolonne durchlaufen läßt, welche mindestens 10 durch Lochböden voneinander getrennte Kammern aufweist.

5. Verfahren gemäß Anspruch 4, dadurch gekennzeichnet, daß die Geschwindigkeit der in der Kolonne aufsteigenden Gasphase in den Löchern der Lochböden bei 2 bis 20 m/sec und die Geschwindigkeit der aufsteigenden Flüssigphase in den Löchern der Lochböden bei 0,05 bis 0,4 m/sec liegt.

**Claims**

1. Process for the continuous preparation of organic mono- or polyisocyanates by the reaction of the mono- or polyamines corresponding to the mono- or polyisocyanates with phosgene dissolved in an organic solvent at elevated temperatures followed by distillative working up of the resulting reaction mixture, characterised
in that to prepare the starting mixtures, the amine component, optionally dissolved in an inert solvent, and the phosgene solution are brought together in a nozzle (1) by constricting one of the two components in this nozzle (1) and supplying the other component to the stream of the first component in this constriction (3) in several partial streams from the side through a corresponding number of apertures (5) distributed over the circumference of the constriction,
in that a flow velocity of from 1 to 10 m/sec is maintained in the constriction,
in that the total length L of the constriction (3) is at least equal to twice the diameter D of the constriction (3) and the length $L_1$ of the part of the constriction (3) in which the axially moving stream flows until it is brought together with the partial streams of the second component is chosen to be from 0.5 to 2 times the diameter D of the constriction (3),
in that the stream of product resulting from the two streams is subjected to a constriction (3) which remains constant and the length $L_2$ of which is at least equal to the length of that path in which the reaction of the free amine is substantially completed,
in that the output ratio of the stream $\epsilon_A$ carried axially to the stream $\epsilon_S$ supplied laterally is maintained at

$$\frac{c_A}{c_S} = \frac{\rho_A \cdot \overset{\circ}{V}_A \cdot v_A^2}{\rho_S \cdot \overset{\circ}{V}_S \cdot v_S^2} = 0.01 \text{ to } 1.0$$

where
$\rho$    denotes the density,
$\overset{\circ}{V}$    denotes the volume stream and
$v$    denotes the flow velocity,
     in each case with the index A denoting the axially supplied stream and the index S the stream supplied laterally,
in that the cross-section of flow continuously increases behind the constriction (3),
and in that the number i of apertures (5) for the partial streams which are supplied laterally is chosen to be: $2 \leq 1 \leq m$ where m is obtained from the formula:

$$\frac{\pi \cdot D}{m \cdot d} = > 1.1$$

where D denotes the diameter of the constriction (3) and d denotes the diameter of the apertures (5).

**2.** Process according to claim 1, characterised in that the larger volume stream is passed through the constriction (3).

**3.** Process according to claim 1 or claim 2, characterised in that the constriction (3) has a constant diameter D over its whole length.

**4.** Process according to one of the claims 1 to 3, characterised in that the reaction mixture obtained by bringing the amine component together with the phosgene solution is continuously passed from below upwards at an elevated temperature but below 150°C through a reaction column which has at least 10 chambers separated by perforated plates.

**5.** Process according to claim 4, characterised in that the velocity of the gaseous phase ascending in the column is from 2 to 20 m/sec. in the perforations of the perforated plates and the velocity of the ascending liquid phase is from 0.05 to 0.4 m/sec in the perforations of the perforated plates.

**Revendications**

**1.** Procédé pour la préparation en continu de mono- ou polyisocyanates organiques en faisant réagir les mono-ou polyamines correspondant aux mono- ou polyisocyanates, avec du phosgène dissous dans un solvant organique, à des températures élevées et en procédant à un traitement ultérieur par voie de distillation du mélange réactionnel résultant, **caractérisé en ce que,** pour la préparation des mélanges de départ, on achemine conjointement le composant d'amine éventuellement dissous dans un solvant inerte et la solution de phosgène dans une tuyère (1), un des deux composants étant soumis à un rétrécissement dans cette tuyère (1) et l'autre composant étant acheminé latéralement dans ce rétrécissement (3) au courant du premier composant, en plusieurs courants partiels, en passant par un nombre correspondant d'alésages (5) répartis sur la périphérie du rétrécissement (3),
**en ce que,** dans le rétrécissement, on maintient une vitesse d'écoulement de 1 à 10 m/sec, en ce que la longueur totale L du rétrécissement (3) correspond au moins à deux fois le diamètre D du rétrécissement (3) et, pour la longueur $L_1$ du rétrécissement (3) du courant amené en direction axiale jusqu'à l'entraînement conjoint avec les courants partiels du deuxième composant, on choisit une valeur qui correspond de 0,5 à 2 fois le diamètre D du rétrécissement (3), **en ce que** le courant de produit résultant des deux courants est soumis à un rétrécissement (3) qui reste constant, dont la longueur $L_2$ correspond au moins à un tronçon dans lequel la réaction de l'amine libre s'arrête pratiquement, **en ce qu'**on maintient le rapport de puissance du courant $\epsilon_A$ amené en direction axiale au courant $\epsilon_S$ amené latéralement

$$\frac{\epsilon_A}{\epsilon_S} = \frac{\rho_A \cdot \overset{\circ}{V}_A \cdot v_A^2}{\rho_S \cdot \overset{\circ}{V}_S \cdot v_S^2} = 0,01 \text{ à } 1,0$$

où

$\rho$ représente la densité,

$\overset{\circ}{V}$ représente le courant en volume,

$v$ représente la vitesse d'écoulement, et, en fait, chaque fois avec l'indice A du courant amené en direction axiale et chaque fois avec l'indice S du courant amené latéralement,

**en ce que,** au-delà du rétrécissement (3), on élargit en continu la section transversale d'écoulement et **en ce qu'**on choisit le nombre i des alésages (5) pour les courants partiels destinés à être amenés latéralement, entre $2 \leqq i \leqq m$, m dérivant de l'équation

$$\frac{\pi \cdot D}{m \cdot d} = > 1,1$$

où D représente le diamètre du rétrécissement (3) et d représente le diamètre des alésages (5).

2. Procédé selon la revendication 1, **caractérisé en ce qu'**on achemine le courant à plus grand volume à travers le rétrécissement (3).

3. Procédé selon la revendication 1 ou 2, **caractérisé en ce que** le rétrécissement (3) présente, sur sa longueur totale L, un diamètre constant D.

4. Procédé selon l'une quelconque des revendications 1 à 3, **caractérisé en ce qu'**on fait s'écouler le mélange réactionnel obtenu par l'acheminement conjoint du composant d'amine avec la solution de phosgène, en continu, de bas en haut, à travers une colonne réactionnelle, à température élevée qui est toutefois inférieure à 150°C, la colonne réactionnelle présentant au moins 10 chambres séparées l'une de l'autre par des fonds à trous.

5. Procédé selon la revendication 4, **caractérisé en ce que** la vitesse de la phase gazeuse ascendante dans la colonne passant par les trous pratiqués dans les fonds à trous, se situe de 2 à 20 m/sec et la vitesse de la phase liquide ascendante passant à travers les trous pratiqués dans les fonds à trous, se situe de 0,05 à 0,4 m/sec.

FIG. 1

FIG. 2

FIG. 3

12